(19) Europäisches Patentamt / European Patent Office / Office européen des brevets

(11) **EP 3 491 330 B1**

(12) **FASCICULE DE BREVET EUROPEEN**

(45) Date de publication et mention
de la délivrance du brevet:
**20.07.2022 Bulletin 2022/29**

(51) Classification Internationale des Brevets (IPC):
**G02B 27/10** *(2006.01)* **G02B 27/14** *(2006.01)*
**G01B 9/02015** *(2022.01)* **G01B 9/02091** *(2022.01)*

(21) Numéro de dépôt: **17752304.0**

(22) Date de dépôt: **25.07.2017**

(52) Classification Coopérative des Brevets (CPC):
**G01B 9/02015; G01B 9/02091; G02B 27/108;
G02B 27/143**

(86) Numéro de dépôt international:
**PCT/EP2017/068717**

(87) Numéro de publication internationale:
**WO 2018/019808 (01.02.2018 Gazette 2018/05)**

(54) **SYSTÈMES ET PROCÉDÉS D'IMAGERIE INTERFÉRENTIELLE PLEIN CHAMP**

INTERFERENZIELLE VOLLFELDBILDGEBUNGSSYSTEME UND VERFAHREN

FULL-FIELD INTERFERENTIAL IMAGING SYSTEMS AND METHODS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **26.07.2016 FR 1657173**

(43) Date de publication de la demande:
**05.06.2019 Bulletin 2019/23**

(73) Titulaire: **Centre National de la Recherche
Scientifique**
**75016 Paris (FR)**

(72) Inventeurs:
• **AUKSORIUS, Egidijus**
**75019 Paris (FR)**
• **BOCCARA, Albert Claude**
**75006 Paris (FR)**

(74) Mandataire: **Marks & Clerk France
Immeuble "Visium"
22, avenue Aristide Briand
94117 Arcueil Cedex (FR)**

(56) Documents cités:
DE-A1-102007 054 283   JP-A- S62 186 841
US-A1- 2006 114 411   US-A1- 2010 157 308
US-A1- 2013 010 302   US-A1- 2013 235 383
US-A1- 2014 111 772

• WATANABE Y ET AL: "In vivo non-mechanical
scanning grating-generated optical coherence
tomography using an InGaAs digital camera",
OPTICS COMMUNICATIONS, ELSEVIER,
AMSTERDAM, NL, vol. 261, no. 2, 15 mai 2006
(2006-05-15), pages 376-380, XP028081474, ISSN:
0030-4018, DOI: 10.1016/J.OPTCOM.2005.12.030
[extrait le 2006-05-15]

• EGIDIJUS AUKSORIUS ET AL: "Fingerprint
imaging from the inside of a finger with full-field
optical coherence tomography", BIOMEDICAL
OPTICS EXPRESS, vol. 6, no. 11, 1 novembre 2015
(2015-11-01), page 4465, XP055273775, United
States ISSN: 2156-7085, DOI:
10.1364/BOE.6.004465

**Description**

## DOMAINE TECHNIQUE

La présente description concerne des systèmes et procédés d'imagerie interférentielle plein champ. Elle s'applique notamment à l'acquisition d'empreintes digitales et l'imagerie de la peau.

## ETAT DE L'ART

Des techniques d'acquisition d'images par imagerie interférentielle plein champ en lumière incohérente, connue sous le nom d'OCT plein champ ou « FF-OCT » selon l'abréviation de l'expression anglo-saxonne « Full-Field Optical Coherence Tomography », sont décrites pour des applications de microscopie interférentielle et constituent des méthodes non invasives et non destructives, très performantes pour l'acquisition d'images en profondeur de tissus biologiques.

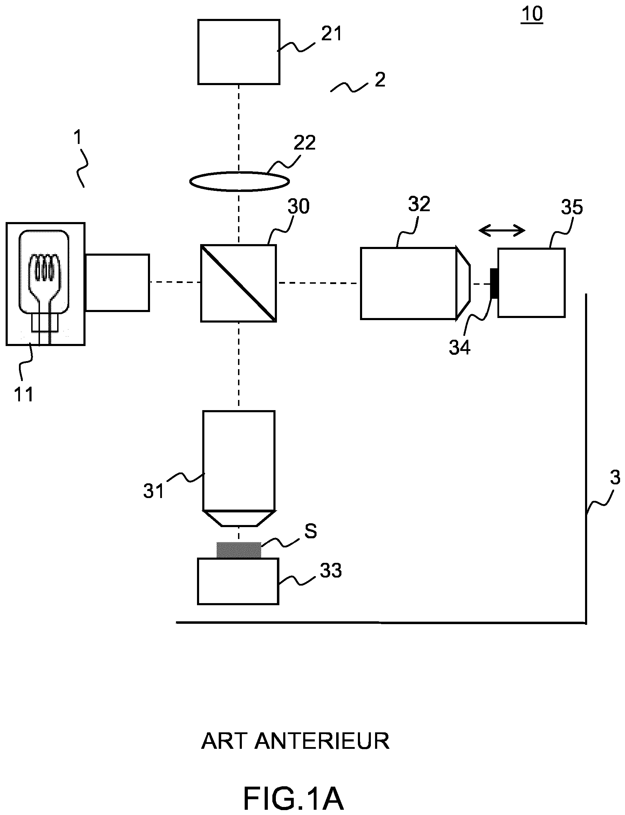

Une technique de microscopie interférentielle plein champ est par exemple décrite dans l'article « Full-field optical coherence tomography » de A. Dubois et C. Boccara, extrait de l'ouvrage « Optical Coherence Tomography - Technology and Applications » - Wolfgang Drexler - James G. Fujimoto - Editors - Springer 2009, dont le montage expérimental est reproduit sur la FIG. 1A.

Le système de microscopie interférentielle 10 connu de l'état de l'art et reproduit sur la FIG. 1A comprend notamment une voie d'illumination 1 avec une source lumineuse 11 de faible cohérence, une voie de détection 2 avec une caméra 21 et une lentille 22, dite lentille de tube, permettant d'assurer la conjugaison optique entre un plan de l'échantillon S que l'on souhaite imager et un plan de détection de la caméra. Le système 10 comprend par ailleurs un interféromètre 3 - dans cet exemple un dispositif de type Linnik - comprenant un élément séparateur 30 adapté pour recevoir les ondes lumineuses émises par la source 11 et les envoyer respectivement vers un bras objet sur lequel est agencé l'échantillon S et un bras de référence sur lequel est agencé un miroir de référence 34. Sur chacun des bras objet et de référence sont positionnés des objectifs de microscope identiques, respectivement référencés 31 et 32 sur la FIG. 1A. Des dispositifs de déplacement axial 33, 35, par exemple des moteurs piézoélectriques, permettent le déplacement respectivement de l'échantillon S et du miroir de référence 34 selon l'axe optique respectivement de l'objectif de microscope 31 et de l'objectif de microscope 32. En pratique, l'échantillon S, par exemple un échantillon de tissu biologique, peut être appliqué contre une fenêtre de silice qui assure la planéité de sa surface et évite ainsi les aberrations que pourraient induire les irrégularités topographiques de la surface de l'échantillon. Pour réduire le signal parasite de réflexion sur la surface de la fenêtre, on peut y déposer un traitement antireflet ou utiliser un objectif à immersion avec un liquide de couplage qui possède un indice de réfraction voisin de celui de la silice.

La technique d'imagerie OCT plein champ est basée sur l'exploitation de la lumière rétrodiffusée par l'échantillon lorsqu'il est éclairé par une source lumineuse à faible longueur de cohérence, et en particulier l'exploitation de la lumière rétrodiffusée par les structures microscopiques cellulaires et tissulaires dans le cas d'un échantillon biologique. Cette technique exploite la faible cohérence temporelle de la source lumineuse pour isoler la lumière rétrodiffusée par une tranche virtuelle en profondeur dans l'échantillon. L'emploi d'un interféromètre permet de générer, par un phénomène d'interférence entre les ondes lumineuses rétrodiffusées par l'échantillon et les ondes lumineuses réfléchies par le miroir de référence, un signal d'interférence représentatif de la lumière provenant sélectivement d'une tranche donnée de l'échantillon, et d'éliminer la lumière provenant du reste de l'échantillon. Cette technique permet d'obtenir des images en trois dimensions avec une résolution typique de l'ordre de $1\mu m$.

Très récemment, E. Auksorius et al. (« Dark-field full-field optical coherence tomography », Optics Letters, Vol. 40, No. 14 (2015)) ont montré comment le contraste des images peut être nettement amélioré dans un système de microscopie interférentielle plein champ du type de celui montré sur la FIG. 1A, grâce à une suppression des réflexions spéculaires sur les interfaces air/verre formées par les fenêtres des éléments de support de l'échantillon notamment.

E. Auksorius et al. : « Fingerprint imaging from the inside of a finger with full-field optical coherence tomography », Biomédical Optics Express, Vol. 6, No. 11 (2015) montre un autre exemple d'un système de microscopie interférentielle plein champ.

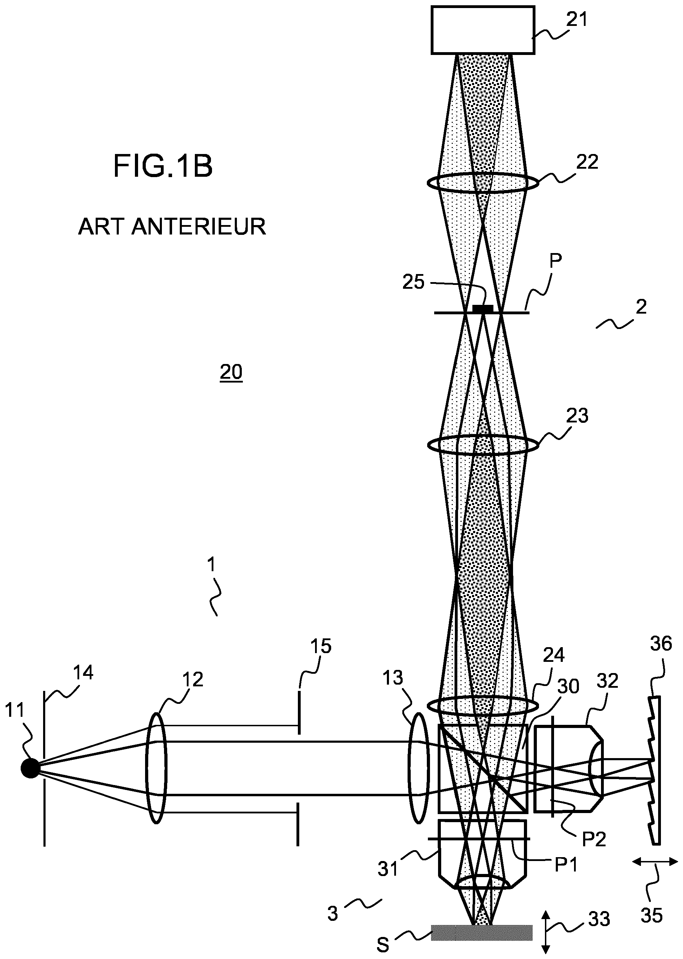

La FIG. 1B représente par un schéma le montage expérimental décrit dans l'article d'E. Auksorius et al. précédemment cité. Le système de microscopie interférentiel 20 représenté sur la FIG. 1B comprend comme celui représenté sur la FIG. 1A une voie d'illumination 1, une voie de détection 2 et un interféromètre 3. L'interféromètre 3 comprend sur chacun des bras objet et de référence formés au moyen de l'élément séparateur 30 un objectif de microscope, respectivement référencés 31 et 32. La voie d'illumination comprend en plus de la source lumineuse 11 un système optique 12, 13 d'imagerie de la source 11 dans un plan focal image des objectifs de microscope. Les plans focaux image des objectifs de microscope 31, 32 sont confondus respectivement avec les plans pupillaires P1, P2 respectivement des objectifs de microscope 31 et 32. Un diaphragme d'ouverture agencé dans un plan 14 sensiblement confondu avec celui de la source 11 permet d'ajuster l'étendue spatiale de la source et un diaphragme de champ agencé dans un plan 15, conjugué avec le miroir de référence, permet d'ajuster le champ de vue sur l'échantillon. La voie de détection 2 comprend en plus de la caméra 21 et de la lentille tube 22 un système optique relai 23, 24, permettant de former un plan pupillaire P conjugué avec les plans pupillaires P1, P2. La suppression des réflexions spéculaires est obtenue au moyen d'un disque opaque 25 positionné sur la voie de détection, dans le plan P conjugué des plans pupillaires P1, P2. Par ailleurs, le miroir de référence sur le bras de référence est remplacé par un réseau à échelettes 36 (ou « blazed grating » selon l'expression anglo-saxonne) de telle sorte que l'ordre zéro est bloqué par le disque opaque, les interférences optiques se formant entre l'ordre 1, dominant par effet de miroitement (« blaze ») diffracté par le réseau et la lumière rétrodiffusée par l'échantillon qui n'est que partiellement obturée par le disque opaque. Les dimensions du diaphragme d'ouverture dans le plan 14 sont ajustées de telle sorte à ce que les dimensions de l'image du diaphragme d'ouverture dans le plan du disque opaque 25 soient plus petites que celles du disque opaque 25 ; ainsi, l'ensemble des réflexions spéculaires issues de l'interféromètre sont bloquées. Un tel système permet de s'affranchir de traitements antireflets sur les fenêtres des éléments de support des échantillons, notamment quand il n'est pas possible de travailler avec des objectifs de microscope à immersion.

Quel que soit le système d'imagerie interférentielle plein champ utilisé, la sensibilité du système est cependant limitée par la quantité de lumière provenant de l'échantillon qui peut être détectée par la caméra. En effet, si R est le coefficient de réflexion de l'élément séparateur 30 et T son coefficient de transmission, et si l'on suppose qu'il n'y a pas de pertes optiques au niveau de l'élément séparateur (R = 1 - T), la quantité de lumière maximale provenant de l'échantillon que l'on peut récupérer est égale à $R \times T = T(1-T)$ et présente un optimum pour $R = T = 0,5$, ce qui correspond à une quantité de lumière maximale provenant de l'échantillon égale à 25% de la quantité de lumière émise par la source.

Une façon possible pour récupérer un flux lumineux plus important en provenance de l'échantillon serait d'augmenter l'intensité lumineuse de la source mais cela n'est pas toujours possible notamment du fait de la limitation du « budget photons » des sources utilisées et du fait de la photosensibilité de certains échantillons. Une autre façon serait de dupliquer la voie de détection pour récupérer à la fois le flux réfléchi et le flux transmis par l'élément séparateur mais cela nécessiterait deux caméras.

La présente description propose une architecture originale pour un système d'imagerie interférentielle plein champ qui permet d'augmenter, par rapport aux systèmes connus de l'état de l'art, la quantité de lumière provenant de l'échantillon qui peut être détectée par la caméra, sans pour autant augmenter le flux lumineux incident sur l'échantillon ou le nombre de caméras. Un tel système d'imagerie interférentielle plein champ est particulièrement bien adapté aux systèmes bas coût pour des applications de type acquisition d'empreintes digitales et imagerie de la peau.

**RESUME**

Selon un premier aspect, la présente description concerne un système d'imagerie interférentielle plein champ d'un échantillon comprenant généralement une voie d'illumination comprenant une source lumineuse pour l'émission d'ondes lumineuses incidentes, un interféromètre, une voie de détection comprenant un dispositif d'acquisition d'images bidimensionnelles et une unité de traitement.

L'interféromètre du système d'imagerie selon le premier aspect comprend au moins un premier objectif, la source lumineuse étant agencée à proximité d'un plan focal image du premier objectif ou à proximité d'un plan conjugué avec un plan focal image du premier objectif et un élément séparateur adapté pour recevoir, par une face d'entrée, les ondes lumineuses incidentes et adapté pour former un bras objet destiné à recevoir l'échantillon et un bras de référence sur lequel est agencé un dispositif de réflexion. L'élément séparateur présente un coefficient de réflexion et un coefficient de transmission non égaux, de telle sorte que la proportion de la puissance optique des ondes lumineuses incidentes envoyées sur le bras objet soit strictement plus grande que la proportion de la puissance optique des ondes lumineuses envoyées sur le bras de référence. Le dispositif de réflexion sur le bras objet est adapté pour renvoyer des ondes lumineuses incidentes dans une direction différente de la direction d'incidence.

Par ailleurs, l'interféromètre du système d'imagerie selon le premier aspect est adapté pour produire, lorsque l'échantillon est disposé sur le bras objet de l'interféromètre, en chaque point d'un champ d'imagerie, une interférence entre une onde de référence obtenue par réflexion d'ondes lumineuses incidentes sur une surface élémentaire du dispositif de réflexion correspondant audit point du champ d'imagerie et une onde objet obtenue par rétrodiffusion d'ondes lumineuses incidentes par un voxel d'une tranche de l'échantillon à une profondeur donnée, ledit voxel correspondant audit point du champ d'imagerie.

Le dispositif d'acquisition d'images bidimensionnelles est positionné dans un plan conjugué du dispositif de réflexion et adapté pour acquérir des signaux interférométriques bidimensionnels résultant des interférences produites en chaque point du champ d'imagerie.

La voie d'illumination et la voie de détection comprennent une voie commune comprenant ladite face d'entrée de l'élément séparateur, et sont séparées par un élément de réflexion, positionné à proximité d'un plan focal image dudit premier objectif, ou à proximité d'un plan conjugué dudit plan focal image du premier objectif, ledit élément de réflexion étant adapté pour laisser passer, sur la voie de détection, au moins une partie des ondes lumineuses renvoyées par le dispositif de réflexion du bras de référence et laisser passer, sur la voie d'illumination, au moins une partie des ondes lumineuses incidentes.

L'unité de traitement est configurée pour calculer une image de l'échantillon à partir desdits signaux interférométriques bidimensionnels.

Les déposants ont montré que cette architecture originale d'un système d'imagerie interférentielle plein champ permettait d'augmenter, par rapport aux systèmes connus de l'état de l'art, la puissance optique rétrodiffusée par l'échantillon et qui peut être détectée par la caméra, sans pour autant augmenter la puissance optique incidente sur l'échantillon ou le nombre de caméras.

Selon un ou plusieurs exemples de réalisation, le coefficient de réflexion et le coefficient de transmission de l'élément séparateur sont tels qu'au moins 90% de la puissance optique des ondes lumineuses incidentes sur l'élément séparateur soient envoyées vers le bras objet. Les déposants ont montré qu'avec une telle configuration, on gagnait plus d'un facteur 3 sur la puissance optique rétrodiffusée par l'échantillon et qui peut être détectée par la caméra, par rapport aux systèmes connus de l'état de l'art.

Selon un ou plusieurs exemples de réalisation, l'élément de réflexion comprend un miroir incliné par rapport à l'axe optique du premier objectif, troué pour le passage des ondes lumineuses incidentes, les dimensions du trou étant suffisamment petites pour ne pas obturer l'ensemble des ondes lumineuses renvoyées sur la voie de détection.

Selon un ou plusieurs exemples de réalisation, l'élément de réflexion comprend un miroir incliné par rapport à l'axe optique du premier objectif pour défléchir vers la voie commune les ondes lumineuses incidentes, et présentant des dimensions suffisamment petites pour ne pas obturer l'ensemble des ondes renvoyées sur la voie de détection.

Dans l'un et/ou l'autre de ces exemples de réalisation, dans le cas d'une source lumineuse incohérente spatialement, d'étendue géométrique donnée, on pourra choisir comme dimension maximale pour le trou - ou pour le miroir incliné - une dimension inférieure ou sensiblement égale à la dimension maximale de l'étendue géométrique de la source rapportée au niveau du miroir.

Selon un ou plusieurs exemples de réalisation, le dispositif de réflexion du bras de référence comprend un réseau de diffraction en réflexion, par exemple un réseau à échelettes.

Selon un ou plusieurs exemples de réalisation, le dispositif de réflexion du bras de référence comprend un miroir incliné.

Selon un ou plusieurs exemples de réalisation, l'interféromètre est un interféromètre de type Michelson, ledit premier objectif étant agencé sur la voie commune des voies d'illumination et de détection.

Selon un ou plusieurs exemples de réalisation, l'interféromètre est un interféromètre de type Linnik et comprend ledit premier objectif sur le bras de référence de l'interféromètre, et un second objectif sur le bras objet de l'interféromètre.

Selon un ou plusieurs exemples de réalisation, lesdits objectifs sont des objectifs de microscope.

Selon un deuxième aspect, la présente description comprend un procédé d'imagerie interférentielle plein champ d'un échantillon au moyen d'un système d'imagerie selon le premier aspect.

Le procédé comprend selon un ou plusieurs exemples de réalisation :

- la mise en place de l'échantillon sur le bras objet de l'interféromètre ;
- la production, au moyen dudit interféromètre, pour chaque point d'un champ d'imagerie, d'une interférence entre une onde de référence obtenue par réflexion d'ondes lumineuses incidentes sur le dispositif de réflexion du bras de référence de l'interféromètre, ladite surface élémentaire correspondant audit point du champ d'imagerie, et une onde objet obtenue par rétrodiffusion de l'onde incidente par un voxel d'une tranche de l'échantillon à une profondeur donnée, ledit voxel correspondant audit point du champ d'imagerie ;
- l'acquisition, pour au moins une valeur de la différence de marche entre le bras objet et le bras de référence, d'au moins un signal interférométrique bidimensionnel résultant des interférences pour chaque point du champ d'imagerie ;
- le calcul d'une image d'une image de l'échantillon à partir desdits signaux interférométriques bidimensionnels.

Les avantages énoncés pour le système d'imagerie sont transposables au procédé d'imagerie selon le deuxième aspect de la présente description.

Les différents modes de réalisation du procédé d'imagerie selon le deuxième aspect de la présente description sont combinables entre eux.

Différentes caractéristiques et modes de réalisation des différents aspects de la présente description peuvent également être combinés entre eux.

**BREVE DESCRIPTION DES FIGURES**

D'autres avantages et caractéristiques de la technique d'imagerie présentée ci-dessus apparaîtront à la lecture de la description détaillée ci-dessous, faite par référence aux figures dans lesquelles :

- la FIG.1A (déjà décrite) illustre un premier système de microscopie interférentielle plein champ selon l'état de l'art;
- La FIG. 1B (déjà décrite) illustre un deuxième système de microscopie interférentielle plein champ selon l'état de l'art;
- les FIGS. 2A et 2B représentent des schémas de principe d'exemples de réalisation d'un système d'imagerie interférentielle plein champ selon un premier mode la présente description;

- les FIGS. 3A et 3B illustrent par des schémas la réflexion d'une onde lumineuse sur un dispositif de réflexion de type réseau (FIG. 3A) et sur un dispositif de réflexion de type miroir (FIG. 3B) ;
- les FIGS.4 et 5 représentent des schémas de principe d'exemples de réalisation d'un système d'imagerie interférentielle plein champ selon un deuxième mode de la présente description;
- la FIG. 6 montre une image expérimentale d'une coupe d'un élément de peau obtenu *in vivo* au moyen d'un système d'imagerie interférentielle plein champ tel que représenté sur la FIG. 2B.

**DESCRIPTION DETAILLEE**

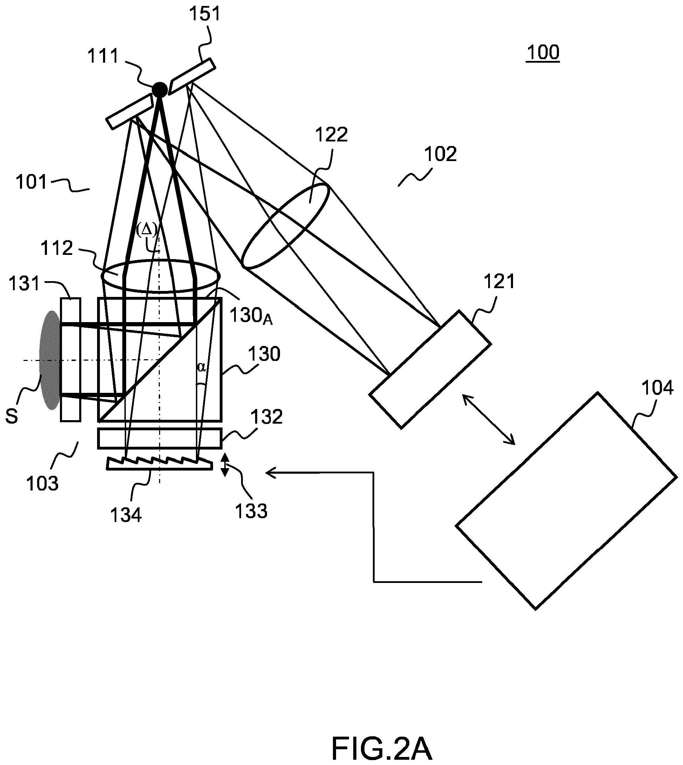

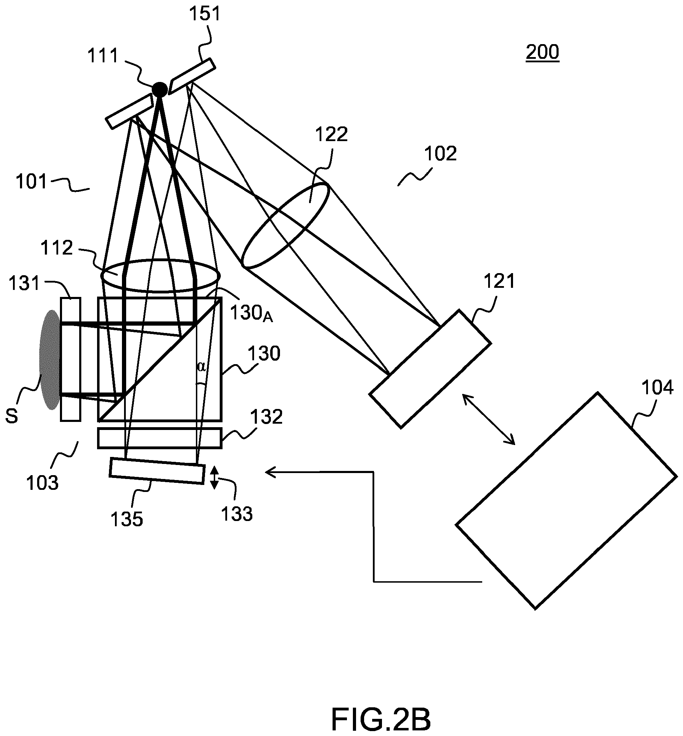

Deux exemples de systèmes d'imagerie interférentielle 100, 200 adaptés à la mise en œuvre de procédés d'imagerie d'un échantillon S selon la présente description sont représentés schématiquement sur les FIGS. 2A et 2B.

Dans chacun de ces exemples, le système d'imagerie interférentielle comprend une voie d'illumination 101 avec notamment une source lumineuse 111 pour l'émission d'ondes lumineuses incidentes, une voie de détection 102 comprenant notamment un dispositif d'acquisition d'images bidimensionnelles 121 et un interféromètre 103 comprenant au moins un premier objectif, dans cet exemple un objectif référencé 112, et un élément séparateur 130 adapté pour recevoir par une face d'entrée $130_A$ les ondes lumineuses incidentes. L'élément séparateur 130 est adapté pour former un bras objet destiné à recevoir l'échantillon S pour lequel on souhaite produire une image, par exemple une image tomographique à une profondeur donnée, et un bras de référence sur lequel est agencé un dispositif de réflexion (134 sur la FIG. 2A et 135, FIG. 2B). Sur le bras de référence, le dispositif de réflexion peut être monté sur un dispositif de déplacement axial symbolisé par une double flèche 133, par exemple un moteur piézoélectrique, un moteur pas à pas ou une combinaison des deux. Le dispositif de déplacement axial permet d'assurer un déplacement axial du dispositif de réflexion, c'est-à-dire un déplacement selon l'axe optique défini par l'axe optique Δ de l'objectif 112 et représenté en pointillés alternés sur la FIG. 2A, et permet notamment de modifier la différence de marche entre les bras objet et de référence. Sur le bras objet, l'échantillon S est par exemple appliqué contre une fenêtre 131 transparente pour les ondes lumineuses incidentes, par exemple une fenêtre en silice. L'échantillon S peut, selon un ou plusieurs exemples de réalisation, être monté également sur un dispositif de déplacement axial (non représenté sur les FIGS. 2A et 2B), par exemple un moteur piézoélectrique, un moteur pas à pas ou une combinaison des deux pour assurer un déplacement axial de l'échantillon et ajuster une profondeur à laquelle on souhaite former une image. Alternativement, le dispositif de déplacement axial 133 pour le déplacement axial du dispositif de réflexion peut assurer cette fonction. Le système d'imagerie interférentielle comprend en outre une unité de traitement 104 relié d'une part au dispositif d'acquisition d'images bidimensionnelles 121 et d'autre part au(x) dispositif(s) mobile(s).

Selon un exemple de réalisation, l'interféromètre 103 est adapté pour produire des interférences optiques entre, d'une part, des ondes de référence obtenues par réflexion de la lumière émise par la source lumineuse 111 par chaque surface élémentaire de la surface du dispositif de réflexion 134, 135 du bras de référence de l'interféromètre et, d'autre part, des ondes objet obtenues par rétrodiffusion de la lumière émise par la même source par chaque voxel d'une tranche de échantillon S en profondeur dans l'échantillon, l'échantillon S étant disposé sur le bras objet de l'interféromètre, ledit voxel et ladite surface élémentaire correspondant au même point du champ d'imagerie.

La source lumineuse 111 est selon un ou plusieurs exemples de réalisation une source incohérente temporellement ou à faible longueur de cohérence temporelle (présentant avantageusement une longueur de cohérence comprise entre 1 et 100 μm). Il s'agit par exemple d'une lampe halogène ou d'une LED (abréviation de l'expression « Light Emitting Diode »).

Le dispositif d'acquisition 121 permet l'acquisition d'au moins un signal interférométrique bidimensionnel résultant des interférences entre les ondes de référence et les ondes objet. Le dispositif d'acquisition 121 est par exemple un capteur d'image, de type caméra CCD (Charge-Coupled Device) ou CMOS (Complementarity metal-oxide-semiconductor). Un tel dispositif d'acquisition est capable d'acquérir des images à cadence élevée, par exemple à une fréquence supérieure à quelques centaines de Hz, voire supérieure à 1 kHz.

L'unité de traitement 104 est configurée pour exécuter au moins une étape de traitement d'au moins un signal interférométrique bidimensionnel acquis par le dispositif d'acquisition 121 et/ou au moins une étape de génération d'image conformément à au moins un des procédés d'imagerie selon la présente description, afin de générer au moins une image de la tranche de l'échantillon S.

Dans un mode de réalisation, l'unité de traitement 104 est un dispositif informatique comprenant une première mémoire CM1 (non représentée) pour le stockage d'images numériques, une deuxième mémoire CM2 (non représentée) pour le stockage d'instructions de programme ainsi qu'un processeur de données, apte à exécuter des instructions de programme stockées dans cette deuxième mémoire CM2, notamment pour commander l'exécution d'au moins une étape de traitement d'au moins un signal interférométrique bidimensionnel acquis par le dispositif d'acquisition 121 et/ou d'au moins une étape de calcul d'image conformément à au moins un des procédés d'imagerie selon la présente description. L'unité de traitement peut également être réalisée sous forme de circuit intégré, comprenant des composants électroniques adaptés pour mettre en œuvre la ou les fonctions décrites dans ce document pour l'unité de traitement. L'unité de traitement 104 peut également être mise en œuvre par un ou plusieurs dispositifs physiquement distincts.

L'interféromètre 103 représenté sur les FIGS. 2A et 2B est un interféromètre de type Michelson et comprend un objectif 112, la source lumineuse 111 étant agencé à proximité d'un plan focal image de l'objectif 112. Ainsi, les ondes lumineuses incidentes émises par la source 111 et issues de l'objectif 112 se trouvent sensiblement collimatées lorsqu'elles se trouvent incidentes sur la face d'entrée $130_A$ de l'élément séparateur 130. Selon un ou plusieurs exemples de réalisation, la source lumineuse 111 peut être agencée à proximité d'un plan conjugué avec un plan focal image de l'objectif 112 auquel cas la voie d'illumination comprend un système optique de transport d'image de la source dans le plan focal image de l'objectif.

Comme illustré sur les FIGS. 2A et 2B, l'élément séparateur 130 présente un coefficient de réflexion et un coefficient de transmission non égaux, de telle sorte que la puissance optique des ondes lumineuses incidentes (plus simplement appelée « puissance optique incidente » dans la suite de la description) envoyée sur le bras objet soit strictement plus grande que la puissance optique des ondes lumineuses incidentes envoyées sur le bras de référence. Dans les exemples des FIGS. 2A et 2B, le coefficient de réflexion pour les ondes lumineuses incidentes est strictement plus grand que le coefficient de transmission de telle sorte qu'une plus grande partie de la puissance optique incidente sur la face d'entrée soit réfléchie vers le bras objet dans lequel se trouve l'échantillon S. Alternativement, le coefficient de transmission peut être strictement plus grand que le coefficient de réflexion auquel cas sur les FIGS 2A et 2B, les positions des bras objet et de référence se trouveraient inversées.

Selon un ou plusieurs exemples de réalisation, le coefficient de réflexion et le coefficient de transmission de l'élément séparateur sont tels qu'au moins 75%, avantageusement au moins 90% de la puissance optique incidente sur l'élément séparateur soit envoyée vers le bras objet.

Ainsi, comme illustré sur les FIGS. 2A et 2B, la voie de détection 102 comprend avec la voie d'illumination 101 une voie commune qui comprend en plus des bras objet et de référence, ladite face d'entrée $130_A$ de l'élément séparateur 130. Les voies de détection et d'illumination sont séparées par un élément de réflexion 151 positionné à proximité d'un plan focal image de l'objectif 112 ou à proximité d'un plan conjugué du plan focal image de l'objectif 112. Dans les exemples des FIGS 2A et 2B, l'élément de réflexion est positionné dans un plan sensiblement confondu avec celui de la source d'émission 111.

Plus précisément dans ces exemples, l'élément de réflexion 151 est un miroir troué qui permet de laisser passer, sur la voie de détection, au moins une partie des ondes lumineuses renvoyées par le dispositif de réflexion du bras de référence et laisser passer, sur la voie d'illumination, au moins une partie des ondes lumineuses émises par la source d'émission 111. Pour ce faire, le dispositif de réflexion du bras de référence est adapté pour renvoyer des ondes lumineuses incidentes dans une direction différente de la direction d'incidence des ondes d'incidence, comme cela sera décrit plus en détails par la suite. Ainsi dans cet exemple, les ondes lumineuses émises par la source d'émission 111 passent à travers le trou tandis que les ondes réfléchies par le dispositif de réflexion du bras de référence sont renvoyées vers le dispositif d'acquisition 121 de la voie de détection.

Selon un ou plusieurs exemples de réalisation, la source lumineuse 111 présente une faible cohérence temporelle mais est cohérente spatialement. Par exemple la source lumineuse 111 est une diode super luminescente, un laser Titane-Saphir à blocage de mode, un laser à fibre à supercontinuum (basée sur la génération d'un supercontinuum dans une fibre non linéaire, par exemple une fibre à cristal photonique). Dans ce cas, le tache de focalisation des ondes lumineuses incidentes étant très petite, le trou dans le miroir 151 peut être très petit, typiquement de dimension transversale maximale (par exemple la dimension du diamètre dans le cas d'un trou rond) comprise entre 5 $\mu$m et 100 $\mu$m, et les pertes du signal rétrodiffusé dues au trou négligeable. Cependant, des sources cohérentes spatialement risquent d'introduire des effets de diaphonie (ou « cross-talk » selon l'expression anglo-saxonne).

Selon un ou plusieurs exemples de réalisation, la source lumineuse 111 présente une faible cohérence temporelle et est incohérente spatialement. Il s'agit par exemple d'une diode électroluminescente (ou « LED »), d'une lampe halogène, ou d'une lampe à arc. On s'affranchit alors des effets de cross-talk. Dans ce cas, le trou du miroir troué 151 est dimensionné pour couvrir une étendue géométrique la plus grande possible de la source - afin de bénéficier de la plus grande puissance optique possible de la source - sans perdre trop du flux rétrodiffusé. Ainsi typiquement, on peut choisir comme dimension maximale pour le trou (par exemple le diamètre), une dimension inférieure ou sensiblement égale à la dimension maximale de l'étendue géométrique de la source au niveau du trou, par exemple une dimension comprise entre 1 mm et 5 mm en fonction de l'étendue spatiale de la source. Par exemple, une LED présente généralement une surface d'émission de dimensions 1 mm $\times$ 1 mm. Cependant, les ondes lumineuses émises sont très divergentes et on peut être amené à agrandir la surface d'émission, par exemple jusqu'à 4 mm $\times$ 4 mm pour réduire la divergence, de telle sorte que toute la lumière puisse être collecté par l'objectif 112. On pourra alors choisir un trou de dimensions correspondantes, par exemple un trou circulaire de diamètre de l'ordre de 4 mm.

L'utilisation d'un élément séparateur 130 avec un coefficient de réflexion et un coefficient de transmission différents permet, grâce à l'agencement des voies d'illumination et de détection précédemment décrits, d'aller au-delà de la quantité maximale provenant de l'échantillon égale à 25% de la quantité de lumière émise par la source, identifiée dans l'art antérieur.

En effet, si R est le coefficient de réflexion de l'élément séparateur 130 et T son coefficient de transmission, et si l'on suppose qu'il n'y a pas de pertes optiques au niveau de l'élément séparateur, la puissance lumineuse maximale provenant de l'échantillon que l'on peut récupérer est maintenant égale à $R \times R$ sur le bras objet et $T \times T$ sur le bras de référence en supposant le cas où $R > T$ (comme sur les FIGS. 2A, 2B). Ainsi par exemple, si $R = 90\%$ et $T = 10\%$, et si l'on fait l'hypothèse que le dispositif de réflexion 134 renvoie 100% de la lumière incidente, il en découle que 1% de la lumière sera transmise après double passage dans l'élément séparateur sur le bras de référence. C'est une proportion de lumière optimale pour l'imagerie de la plupart des tissus biologiques, car on cherche à avoir sensiblement la même quantité de lumière renvoyée par le dispositif de réflexion et par l'échantillon. Si l'on suppose que l'échantillon S renvoie 100% de la lumière incidente, il en découle que la puissance optique maximale provenant de l'échantillon que l'on peut récupérer est plus de 3 fois supérieure à la puissance optique la quantité de lumière que l'on obtenait avec un élément séparateur 50/50 tel que décrit dans l'état de l'art.

Dans l'exemple de la FIG. 2A, le dispositif de réflexion 134 est un réseau de diffraction en réflexion, par exemple un réseau à échelettes (ou « blazed grating » selon l'expression anglo-saxonne), les interférences optiques se formant entre l'ordre 1, dominant par effet de miroitement (« blaze ») diffracté par le réseau, et la lumière rétrodiffusée par l'échantillon.

Pour dimensionner le réseau, on détermine tout d'abord l'angle $\alpha$ qui permet de renvoyer la lumière diffractée par le réseau en dehors du trou. Si f est la focale de l'objectif 112 et D est le diamètre du trou dans le miroir troué 151, l'angle vérifie $\alpha$:

$$\alpha = \text{Arctan}\,(D/f) \qquad (1)$$

On choisit ensuite un réseau qui permet de diffracter les ondes incidentes avec l'angle $\alpha$. On peut par exemple utiliser la formule des réseaux:

$$d = \lambda/\sin(\alpha) \qquad (2)$$

Où d est la distance entre deux traits du réseau et $\lambda$ est la longueur d'onde.

Ainsi par exemple, pour une focale f = 100 mm, un diamètre D = 4 mm, on calcule $a$ = 2.3°. On peut en déduire d = $\lambda/\sin(\alpha) \approx \lambda \times f/D = 0.85\mu m \times 100mm/4mm = 21,25\ \mu m$, ou, en terme de densité de lignes, 47 lignes par millimètre (« lpm »).

D'autres dispositifs de réflexion qu'un réseau de diffraction sont possibles.

Ainsi, dans l'exemple de la FIG. 2B, le dispositif de réflexion 135 comprend un simple miroir incliné, adapté également à renvoyer des ondes lumineuses incidentes dans une direction différente de la direction d'incidence des ondes d'incidence. Dans ce cas, l'inclinaison du miroir 135 doit être choisie de la même manière pour que les ondes réfléchies par le miroir ne soient pas renvoyées dans le trou. Pour cela, on choisira par exemple un miroir incliné avec un angle égal à $a$ /2 par rapport à l'axe optique de l'objectif 112.

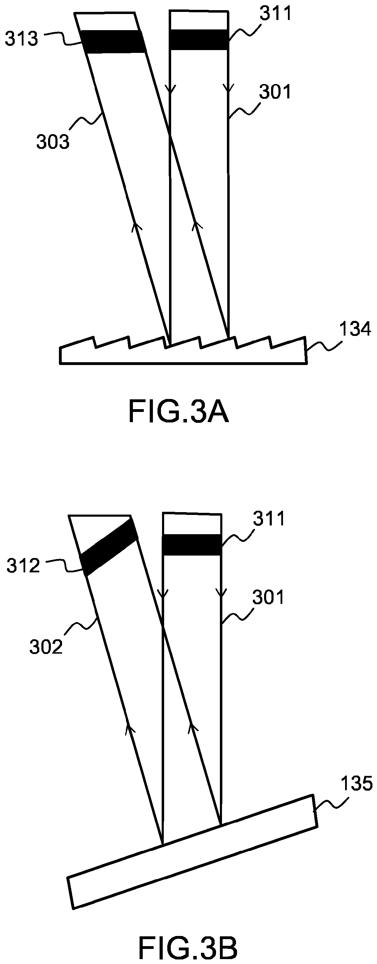

En pratique, dans les systèmes d'imagerie interférentielle selon la présente description, on pourra préférer l'utilisation d'un réseau de diffraction ou celle d'un miroir incliné comme dispositif de réflexion, pour les raisons expliquées en référence aux FIGS 3A et 3B.

Le réseau de diffraction peut être utilisé par exemple dans les cas où l'on souhaite des images *en face* d'un échantillon ; en effet, comme illustré sur la FIG. 3A, l'orientation de la tranche de cohérence 313 du faisceau 303 difracté par le réseau 134 sera la même que l'orientation de la tranche de cohérence 311 du faisceau 301 incidente sur le réseau. Comme la lumière rétrodiffusée par l'échantillon conserve également sa tranche de cohérence parallèle au faisceau incident, elle va interférer avec le faisceau de référence en face - à travers tout le champ de vision - et donc, va produire une image en face. Par exemple, une imagerie *en face* est préférée dans l'imagerie d'empreintes digitales puisque les empreintes digitales internes se trouvent dans cette orientation.

Dans le cas de l'utilisation d'un miroir incliné (135, FIG. 3B), la tranche de cohérence 312 du faisceau réfléchi est inclinée par rapport à la tranche de cohérence 311 du faisceau incident 301. Ainsi, quand un tel faisceau de référence interfère avec des faisceaux rétrodiffusés par un échantillon, une image est générée à un angle non nul par rapport à la situation *en face* précédemment décrite. Cette imagerie peut être utilisée lorsque l'on veut acquérir des images avec une coupe inclinée par rapport à la surface ou lorsque différentes couches orientées parallèlement à la surface doivent être capturées dans une seule image.

Dans l'exemple des FIGS. 2A et 2B, est par ailleurs placée sur la voie de détection 102 une optique 122, appelée dans la suite de la description « lentille intermédiaire », par exemple un doublet achromatique, qui permet de conjuguer le plan focal objet de l'objectif 112 et le plan de détection du dispositif d'acquisition 121 pour permettre l'acquisition des signaux d'interférence produits par le interféromètre par le dispositif d'acquisition. La focale de la lentille intermédiaire est adaptée pour permettre un échantillonnage adéquat de l'échantillon S par le dispositif d'acquisition 121. La focale de l'optique 122 est par exemple comprise entre 30 mm et 300 mm, par exemple atour d'une centaine de millimètres.

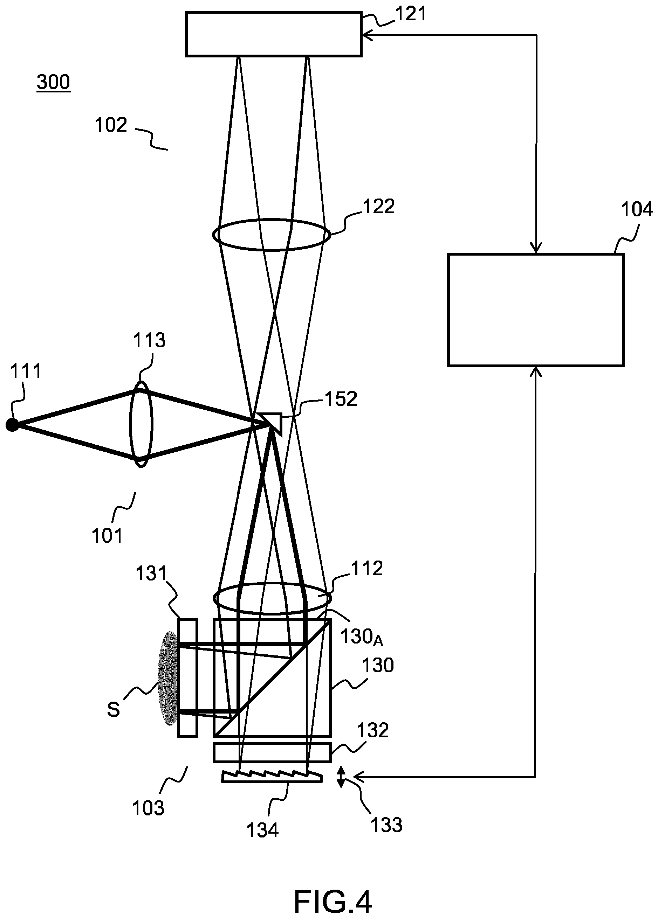

La FIG. 4 illustre un autre mode de réalisation d'un système d'imagerie interférentiel, semblable à celui représenté sur la FIG. 2A, amis dans lequel l'élément de réflexion comprend un petit miroir 152 incliné par rapport à l'axe optique de l'objectif 112 pour défléchir vers la voie commune les ondes lumineuses incidentes issues par la source lumineuse 111. Le miroir 152 est agencé dans un plan focal image de l'objectif 112. Les dimensions du miroir 152 sont suffisamment petites pour ne pas obturer l'ensemble des ondes renvoyées sur la voie de détection. En pratique, le dimensionnement du miroir 152 répond aux mêmes contraintes que celles décrites pour le trou du miroir troué 151. Ainsi, une dimension maximale du miroir peut être selon un exemple inférieur ou sensiblement égal à une dimension maximale de l'étendue géométrique de la source, rapportée dans le plan du miroir.

Dans cet exemple de réalisation, comme cela est montré sur la FIG. 4, la voie d'illumination 101 comprend un système optique 113 de transport d'image de la source 111 dans le plan focal image de l'objectif 112.

Dans l'exemple de la FIG. 4, le dispositif de réflexion 134 est un réseau de diffraction en réflexion ; bien entendu, un système d'imagerie interférentielle similaire pourrait être obtenu en remplaçant le réseau de diffraction en réflexion par un miroir incliné, comme cela a été décrit précédemment.

Dans l'exemple des systèmes d'imagerie interférentielle représentés sur les FIGS. 2A, 2B, 4, l'interféromètre est de type interféromètre de Michelson et comprend un objectif 112 agencé en amont de l'élément séparateur 130. Bien entendu, d'autres types d'interféromètres peuvent être envisagés pour les systèmes d'imagerie interférentielle selon la présente description, et notamment des interféromètres de type Linnik.

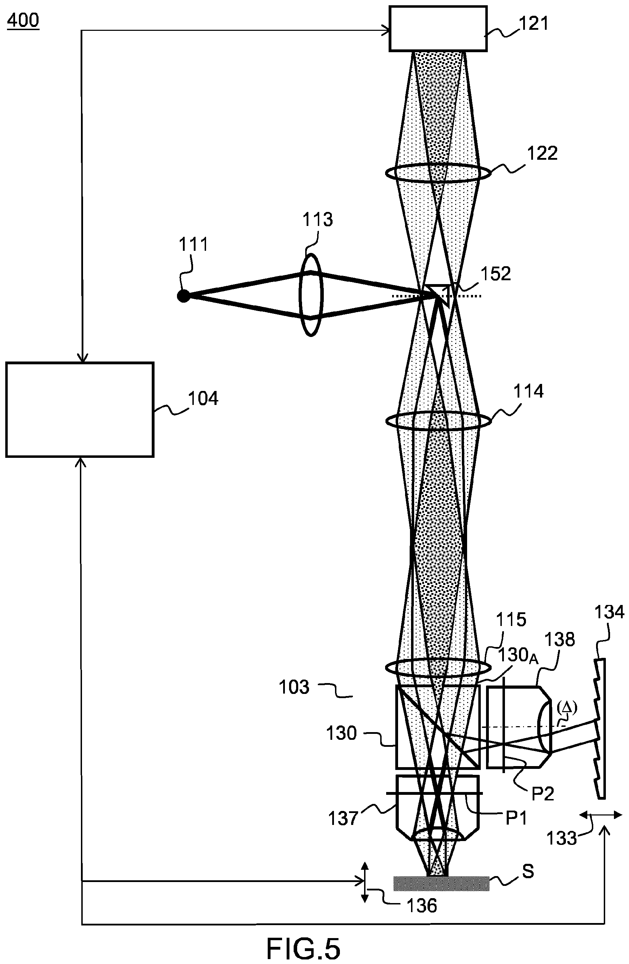

Ainsi, La FIG. 5 représente un exemple de système d'imagerie interférentielle sensiblement similaire à celui représenté sur la FIG. 4 mais comprenant un interféromètre de type Linnik.

L'interféromètre 103 comprend alors un premier objectif 138 sur le bras de référence de l'interféromètre, et un second objectif 137 sur le bras objet de l'interféromètre.

Le dimensionnement de l'ensemble des éléments peut se faire de la même manière que dans les exemples précédentes (FIG. 2A, 2B, 4), mais en considérant le premier objectif 138 en lieu et place de l'objectif 112.

Dans l'exemple illustré sur la FIG. 5, la voie commune à la voie d'illumination 101 et la voie de détection 102 comprend en outre une optique relai 114, 115 permettant de conjuguer l'élément de réflexion, dans cet exemple le miroir 152, avec le plan focal image du premier objectif 138.

Comme cela est visible sur la FIG. 5, dans cet exemple, le bras objet reçoit les ondes lumineuses incidentes transmises par l'élément séparateur 130, le coefficient de transmission de l'élément séparateur 130 étant strictement plus grand que le coefficient de réflexion ; mais le bras objet pourrait dans un autre exemple recevoir les ondes lumineuses incidentes réfléchies par l'élément séparateur 130 dans le cas où le coefficient de réflexion de l'élément séparateur 130 est strictement plus grand que le coefficient de transmission.

Dans les exemples illustrés ci-dessus, l'élément séparateur 130 représenté est un cube séparateur. Bien entendu, tout dispositif adapté pour séparer la puissance optique incidente selon des proportions inégales pourra fonctionner. Par exemple, l'élément séparateur 130 est un cube séparateur à deux prismes, une lame séparatrice pelliculaire ou un réseau de diffraction gravé sur une plaque de verre.

La présente description concerne également des procédés d'imagerie interférentielle plein champ d'un échantillon S au moyen d'un système d'imagerie interférentielle selon la présente description, par exemple un système d'imagerie interférentielle selon l'un des exemples de réalisation décrit précédemment.

Le procédé d'imagerie interférentielle comprend tout d'abord la mise en place d'un échantillon S sur le bras objet de l'interféromètre 103. La mise en place de l'échantillon S dépend de l'application recherchée. Par exemple, l'échantillon peut être une zone de la peau d'un être humain, par exemple la peau du doigt pour l'acquisition d'empreintes digitales en surface ou en profondeur dans le doigt, auquel cas le doigt pourra être plaqué contre une fenêtre transparente, référencée 131 sur les figures. Dans le cas d'une application au diagnostic de la peau, la taille de la fenêtre sera réduite au minimum, par exemple un cercle de 5 à 10 mm de diamètre et la fenêtre sera placée à l'extrémité d'un tube afin d'accéder facilement à différentes zones du corps humain (visage, aisselles). Notons que le montage étant compact et léger il pourra aisément être placé au bout d'un bras articulé.

Dans le cas d'une application à la détection de défauts sur des surfaces semi-conductrices, une grande surface plane au contraire sera nécessaire pour y déposer les surfaces.

Le procédé d'imagerie interférentielle selon la présente description comprend la production, au moyen de l'interféromètre 103, pour chaque point du champ d'imagerie souhaité, d'une interférence entre une onde de référence obtenue par réflexion d'ondes lumineuses incidentes sur le dispositif de réflexion du bras de référence de l'interféromètre, ladite surface élémentaire correspondant audit point du champ d'imagerie, et une onde objet obtenue par rétrodiffusion de l'onde incidente par un voxel d'une tranche de l'échantillon à une profondeur donnée, ledit voxel correspondant audit point du champ d'imagerie. Est alors acquis, pour au moins une valeur de la différence de marche entre le bras objet et le bras de référence, au moins un signal interférométrique bidimensionnel résultant des interférences pour chaque point du champ d'imagerie, puis il est procédé au calcul d'une image de l'échantillon à partir desdits signaux interférométriques bidimensionnels.

Plus précisément, lorsque la source de lumière 111 présente une faible longueur de cohérence temporelle, des interférences entre la lumière réfléchie par la surface de réflexion (134, 135) (onde de référence) et celle rétrodiffusée par l'échantillon S n'ont lieu que lorsque les chemins optiques dans les deux bras sont égaux, à la longueur de cohérence près. Ainsi, des interférences ont lieu entre l'onde de référence et la lumière rétrodiffusée par chaque voxel d'une tranche située dans un plan perpendiculaire à l'axe optique du bras objet, à une profondeur donnée de l'échantillon, appelée tranche de cohérence, un voxel étant un volume élémentaire défini dans la tranche de cohérence. La lumière rétrodiffusée par chaque voxel est représentative de l'amplitude de la somme cohérente des ondes rétrodiffusées par l'ensemble des structures élémentaires diffusantes présentes dans ce voxel.

Les signaux interférométriques résultant des interférences optiques entre les ondes de référence et les ondes rétrodiffusées par les différents voxels sont acquis en parallèle à un instant t par le dispositif d'acquisition 121. Il en résulte une image interférométrique S correspondant à l'état d'interférence à un instant t donné de la tranche de cohérence. Un élément d'image ou pixel d'image interférométrique situé à une position donnée (x,y), définie relativement à un repère bidimensionnel associé au dispositif d'acquisition 121, présente une valeur S(x,y,t) qui correspond à l'intensité du signal interférométrique, acquis à l'instant t à la position (x,y), résultant de l'interférence entre l'onde rétrodiffusée par le voxel de position correspondante dans l'échantillon et l'onde de référence réfléchie par une surface élémentaire de la surface de réflexion du bras de référence de position correspondante.

L'unité de traitement 104 est configurée pour générer une image de l'échantillon S à partir d'au moins signal interférométrique bidimensionnel obtenu par le dispositif d'acquisition 121.

Différents procédés connus de l'état de l'art permettent le calcul d'une image de l'échantillon. Par exemple, une méthode par modulation à 4 phases est décrite dans l'article « Full-field optical coherence tomography » de A. Dubois et C. Boccara, extrait de l'ouvrage « Optical Coherence Tomography - Technology and Applications » - Wolfgang Drexler - James G. Fujimoto - Editors - Springer 2009. Selon cette méthode, des signaux interférentiels sont acquis par le dispositif d'acquisition pour 4 valeurs de déphasage et l'image de l'échantillon est reconstruite par calcul de la racine carrée de $(I_0-I_{180})^2 + (I_{90}-I_{270})^2$, où $I_0$, $I_{90}$, $I_{180}$, $I_{270}$ représentent des signaux interférentiels acquis pour 4 valeurs de déphasage.

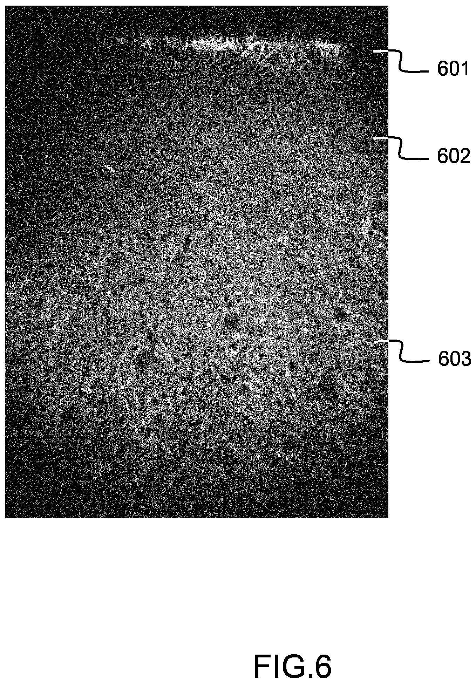

La FIG. 6 montre une image expérimentale d'un échantillon de peau, vu dans la profondeur, et obtenu par un système d'imagerie interférentielle plein champ selon la présente description, plus précisément un système d'imagerie interférentielle plein champ tel que représenté sur la FIG. 2B.

Le système comprend une source 111 formée d'une LED émettant une lumière incohérente à une longueur d'onde de 850 nm (DEL M850L3, Thorlabs®), avec une largeur spectrale de 30 nm. Le système comprend un interféromètre 103 de type Michelson et une caméra 121 de type CMOS (Adimec® CXP) pouvant fonctionner jusqu'à 700 images par seconde. L'étendue géométrique de la source lumineuse est agrandie par un facteur 4 au moyen d'une une paire de lentilles (non représentée sur la FIG. 2B) afin de diminuer la divergence de la lumière émergeant de la LED, de telle sorte à ce que la majeure partie de la lumière émise se trouve dans l'angle d'acceptation de l'objectif 112. La LED est focalisée au niveau du trou du miroir 151, circulaire, de 4 mm de diamètre. Un dispositif de réflexion formé d'un miroir 135 incliné est utilisé, l'angle d'inclinaison étant de 1,15 degré de telle sorte à ce que la lumière réfléchie soit réfléchie par le miroir 151 vers la voie de détection 102. Un élément piézoélectrique 133 est utilisé pour moduler le motif d'interférence détecté par la caméra. Des images sont acquises pour 4 valeurs de déphasage et l'image de l'échantillon est reconstruite par calcul de la racine carrée de $(I_0-I_{180})^2 + (I_{90}-I_{270})^2$, où $I_0$, $I_{90}$, $I_{180}$, $I_{270}$ représentent des signaux interférentiels acquis pour 4 valeurs de déphasage.

Sur la FIG. 6, on observe une zone 601 en surface de la peau, une zone 602 en-dessous de la surface et une zone 603 en profondeur.

Dans cet exemple, l'utilisation d'un miroir incliné 135 permet d'obtenir des informations en profondeur dans l'échantillon avec une seule image, (sans déplacement axial de l'échantillon ou du miroir de référence, à l'exception du mouvement piézoélectrique qui utilisé pour moduler la phase). Ainsi, on peut estimer que la zone 601 est à une profondeur de 44 $\mu$m, la zone 602 est à une profondeur de123 $\mu$m et la zone 603 est à une profondeur 312 $\mu$m. La différence d'intensité est liée à l'amortissement de la lumière balistique par le tissu biologique.

Les systèmes et procédés d'imagerie interférentielle décrits ci-dessus s'appliquent notamment à l'acquisition d'empreintes digitales ou à l'imagerie de la peau. Ils peuvent également s'appliquer de façon générale dans toutes les situations où le budget photons est limité. Ils sont en outre applicables à des échantillons quelconques, que ces échantillons soient biologiques ou non.

## Revendications

1. Système d'imagerie interférentielle plein champ (100, 200, 300, 400) d'un échantillon (S) comprenant :

    - une voie d'illumination (101) comprenant une source lumineuse (111) pour l'émission d'ondes lumineuses incidentes;

- un interféromètre (103) comprenant :

o au moins un premier objectif (112, 138), la source lumineuse étant agencée à proximité d'un plan focal image du premier objectif ou à proximité d'un plan conjugué avec un plan focal image du premier objectif ; et
o un élément séparateur (130) adapté pour recevoir, par une face d'entrée (130$_A$), les ondes lumineuses incidentes et adapté pour former un bras objet destiné à recevoir l'échantillon (S) et un bras de référence sur lequel est agencé un dispositif de réflexion (134, 135) ; et dans lequel

▪ l'élément séparateur (130) présente un coefficient de réflexion et un coefficient de transmission non égaux, de telle sorte que la proportion de la puissance optique des ondes lumineuses incidentes envoyées sur le bras objet soit strictement plus grande que la proportion de la puissance optique des ondes lumineuses envoyées sur le bras de référence ;
▪ le dispositif de réflexion est adapté pour renvoyer des ondes lumineuses incidentes dans une direction différente de la direction d'incidence, et
▪ l'interféromètre est adapté pour produire, lorsque l'échantillon est disposé sur le bras objet de l'interféromètre, en chaque point d'un champ d'imagerie, une interférence entre une onde de référence obtenue par réflexion d'ondes lumineuses incidentes sur une surface élémentaire du dispositif de réflexion correspondant audit point du champ d'imagerie et une onde objet obtenue par rétrodiffusion d'ondes lumineuses incidentes par un voxel d'une tranche de l'échantillon à une profondeur donnée, ledit voxel correspondant audit point du champ d'imagerie;

- une voie de détection (102) comprenant un dispositif d'acquisition d'images bidimensionnelles (121), et dans laquelle :

o le dispositif d'acquisition d'images bidimensionnelles est positionné dans un plan conjugué du dispositif de réflexion (134, 135) et adapté pour acquérir des signaux interférométriques bidimensionnels résultant des interférences produites en chaque point du champ d'imagerie;
o la voie d'illumination (101) et la voie de détection (102) comprennent une voie commune comprenant ladite face d'entrée de l'élément séparateur (130), et sont séparées par un élément de réflexion (151, 152), positionné à proximité d'un plan focal image dudit premier objectif (112, 138), ou à proximité d'un plan conjugué dudit plan focal image du premier objectif, ledit élément de réflexion étant adapté pour laisser passer, sur la voie de détection, au moins une partie des ondes lumineuses renvoyées par le dispositif de réflexion du bras de référence et laisser passer, sur la voie d'illumination, au moins une partie des ondes lumineuses incidentes ;

- une unité de traitement (104) configurée pour calculer une image de l'échantillon à partir desdits signaux interférométriques bidimensionnels.

2. Système d'imagerie selon la revendication 1, dans lequel le coefficient de réflexion et le coefficient de transmission de l'élément séparateur sont tels qu'au moins 90% de la puissance optique des ondes lumineuses incidentes sur l'élément séparateur soient envoyées vers le bras objet.

3. Système d'imagerie selon l'une quelconque des revendications précédentes, dans lequel l'élément de réflexion comprend un miroir incliné par rapport à l'axe optique du premier objectif, troué pour le passage des ondes lumineuses incidentes, les dimensions du trou étant suffisamment petites pour ne pas obturer l'ensemble des ondes lumineuses renvoyées sur la voie de détection.

4. Système d'imagerie selon l'une quelconque des revendications 1 ou 2, dans lequel l'élément de réflexion comprend un miroir incliné par rapport à l'axe optique du premier objectif pour défléchir vers la voie commune les ondes lumineuses incidentes, et présentant des dimensions suffisamment petites pour ne pas obturer l'ensemble des ondes renvoyées sur la voie de détection.

5. Système d'imagerie selon l'une quelconque des revendications précédentes, dans lequel le dispositif de réflexion comprend un réseau de diffraction en réflexion, par exemple un réseau à échelettes.

6. Système d'imagerie selon l'une quelconque des revendications 1 à 4, dans lequel le dispositif de réflexion comprend un miroir incliné.

7. Système d'imagerie selon l'une quelconque des revendications précédentes, dans lequel l'interféromètre (103) est un interféromètre de type Michelson, ledit premier objectif (112) étant agencé sur la voie commune des voies d'illumination (101) et de détection (102).

8. Système d'imagerie selon l'une quelconque des revendications 1 à 6, dans lequel l'interféromètre (103) est un interféromètre de type Linnik et comprend ledit premier objectif (138) sur le bras de référence de l'interféromètre, et un second objectif sur le bras objet de l'interféromètre.

9. Système d'imagerie selon la revendication 8, dans lequel lesdits objectifs sont des objectifs de microscope.

10. Procédé d'imagerie interférentielle plein champ d'un échantillon (S) au moyen d'un système d'imagerie selon l'une quelconque des revendications 1 à 9 comprenant :

- la mise en place de l'échantillon (S) sur le bras objet de l'interféromètre (103) ;
- la production, au moyen dudit interféromètre, pour chaque point d'un champ d'imagerie, d'une interférence entre une onde de référence obtenue par réflexion d'ondes lumineuses incidentes sur le dispositif de réflexion du bras de référence de l'interféromètre, ladite surface élémentaire correspondant audit point du champ d'imagerie, et une onde objet obtenue par rétrodiffusion de l'onde incidente par un voxel d'une tranche de l'échantillon à une profondeur donnée, ledit voxel correspondant audit point du champ d'imagerie ;
- l'acquisition, pour au moins une valeur de la différence de marche entre le bras objet et le bras de référence, d'au moins un signal interférométrique bidimensionnel résultant des interférences pour chaque point du champ d'imagerie ;
- le calcul d'une image d'une image de l'échantillon à partir desdits signaux interférométriques bidimensionnels.

**Patentansprüche**

1. System zur interferenziellen Vollfeldbildgebung (100, 200, 300, 400) einer Probe (S), das Folgendes umfasst:

- einen Beleuchtungspfad (101) mit einer Lichtquelle (111) zum Emittieren von einfallenden Lichtwellen;
- ein Interferometer (103), das Folgendes umfasst:

o mindestens ein erstes Objektiv (112, 138), wobei die Lichtquelle in der Nähe einer Bildbrennebene des ersten Objektivs oder in der Nähe einer mit einer Bildbrennebene des ersten Objektivs konjugierten Ebene angeordnet ist; und
o ein Trennelement (130), ausgelegt zum Empfangen, über eine Eintrittsfläche (130$_A$), der einfallenden Lichtwellen, und ausgelegt zum Bilden eines Objektarms zum Empfangen der Probe (S), und eines Referenzarms, an dem eine Reflexionsvorrichtung (134, 135) angeordnet ist; und wobei

■ das Trennelement (130) einen Reflexions- und Transmissionskoeffizienten aufweist, die ungleich sind, so dass der Anteil der optischen Leistung der auf den Objektarm gesendeten einfallenden Lichtwellen strikt größer ist als der Anteil der optischen Leistung der auf den Referenzarm gesendeten Lichtwellen;
■ die Reflexionsvorrichtung zum Zurückwerfen der einfallenden Lichtwellen in eine andere Richtung als die Einfallsrichtung ausgelegt ist, und
■ das Interferometer so ausgelegt ist, dass es, wenn die Probe auf dem Objektarm des Interferometers angeordnet ist, an jedem Punkt eines Abbildungsfeldes eine Interferenz zwischen einer Referenzwelle, die durch Reflexion einfallender Lichtwellen an einer dem Punkt des Abbildungsfeldes entsprechenden elementaren Oberfläche der Reflexionseinrichtung erhalten wird, und eine Objektwelle erzeugt, die durch Rückstreuung einfallender Lichtwellen von einem Voxel einer Scheibe der Probe in einer gegebenen Tiefe erhalten wird, wobei das Voxel dem Punkt des Abbildungsfeldes entspricht;

- einen Detektionspfad (102), der eine zweidimensionale Bilderfassungsvorrichtung (121) umfasst, und wobei:

o die zweidimensionale Bilderfassungsvorrichtung in einer konjugierten Ebene der Reflexionsvorrichtung (134, 135) positioniert und zum Erfassen zweidimensionaler interferometrischer Signale ausgelegt ist, die aus den an jedem Punkt des Abbildungsfeldes erzeugten Interferenzen resultieren;
o der Beleuchtungspfad (101) und der Detektionspfad (102) einen gemeinsamen Pfad umfassen, der die Eintrittsfläche des Trennelements (130) umfasst, und durch ein Reflexionselement (151, 152) getrennt sind, das in der Nähe einer Bildbrennebene des ersten Objektivs (112, 138) oder in der Nähe einer konjugierten Ebene der Bildbrennebene des ersten Objektivs positioniert ist, wobei das Reflexionselement zum Durchlassen mindestens eines Teils der von der Reflexionseinrichtung des Referenzarms zurückgeworfenen Lichtwellen auf den Detektionspfad und zum Durchlassen mindestens eines Teils der einfallenden Lichtwellen auf den Beleuchtungspfad ausgelegt ist;

- eine Verarbeitungseinheit (104), die zum Berechnen eines Bildes der Probe aus den zweidimensionalen interferometrischen Signalen konfiguriert ist.

2. Bildgebungssystem nach Anspruch 1, wobei der Reflexionskoeffizient und der Transmissionskoeffizient des Trennelements derart sind, dass mindestens 90% der optischen Leistung der auf das Trennelement einfallenden Lichtwellen zum Objektarm gesendet werden.

3. Bildgebungssystem nach einem der vorhergehenden Ansprüche, wobei das Reflexionselement einen in Bezug auf die optische Achse des ersten Objektivs geneigten Spiegel umfasst, der für den Durchgang der einfallenden Lichtwellen durchlöchert ist, wobei die Abmessungen des Lochs ausreichend klein sind, um nicht die Gesamtheit der auf den Detektionspfad zurückgeworfenen Lichtwellen zu blockieren.

4. Bildgebungssystem nach einem der Ansprüche 1 oder 2, wobei das Reflexionselement einen in Bezug auf die optische Achse des ersten Objektivs geneigten Spiegel umfasst, um die einfallenden Lichtwellen zu dem gemeinsamen Pfad abzulenken, und ausreichend kleine Abmessungen aufweist, um nicht alle auf den Detektionspfad zurückgeworfenen Wellen zu blockieren.

5. Bildgebungssystem nach einem der vorhergehenden Ansprüche, wobei die Reflexionsvorrichtung ein Reflexionsbeugungsgitter, z.B. ein Blaze-Gitter, umfasst.

6. Bildgebungssystem nach einem der Ansprüche 1 bis 4, wobei die Reflexionseinrichtung einen geneigten Spiegel umfasst.

7. Bildgebungssystem nach einem der vorhergehenden Ansprüche, wobei das Interferometer (103) ein Interferometer vom Michelson-Typ ist, wobei die erste Linse (112) auf dem gemeinsamen Pfad des Beleuchtungspfads (101) und des Detektionspfads (102) angeordnet ist.

8. Bildgebungssystem nach einem der Ansprüche 1 bis 6, wobei das Interferometer (103) ein Interferometer vom Linnik-Typ ist und das erste Objektiv (138) auf dem Referenzarm des Interferometers und ein zweites Objektiv auf dem Objektarm des Interferometers umfasst.

9. Bildgebungssystem nach Anspruch 8, wobei die Objektive Mikroskopobjektive sind.

10. Verfahren zur Vollfeld-Interferenzabbildung einer Probe (S) mit Hilfe eines Bildgebungssystems nach einem der Ansprüche 1 bis 9, das Folgendes umfasst:

- Platzieren der Probe (S) auf dem Objektarm des Interferometers (103);
- Erzeugen, mittels des Interferometers, für jeden Punkt eines Abbildungsfeldes, einer Interferenz zwischen einer Referenzwelle, die durch Reflexion einfallender Lichtwellen an der Reflexionsvorrichtung des Referenzarms des Interferometers erhalten wird, wobei die elementare Oberfläche dem Punkt des Abbildungsfeldes entspricht, und einer Objektwelle, die durch Rückstreuung der einfallenden Welle durch ein Voxel einer Scheibe der Probe in einer gegebenen Tiefe erhalten wird, wobei das Voxel dem Punkt des Abbildungsfeldes entspricht;
- Erfassen, für mindestens einen Wert der Wegdifferenz zwischen dem Objektarm und dem Referenzarm, mindestens eines zweidimensionalen interferometrischen Signals, das sich aus der Interferenz für jeden Punkt des Abbildungsfeldes ergibt;
- Berechnen eines Bildes der Probe aus den zweidimensionalen interferometrischen Signalen.

## Claims

1. A full-field interferential imaging system (100, 200, 300, 400) for imaging a sample (S), comprising:

- an illumination channel (101) comprising a light source (111) for emitting incident light waves;
- an interferometer (103) comprising:

o at least one first objective (112, 138), the light source being arranged in proximity to an image focal plane of the first objective or in proximity to a plane conjugate with an image focal plane of the first objective; and
o a splitting element (130) suitable for receiving, via an entrance face (130$_A$), the incident light waves and suitable for forming an object arm intended to receive the sample (S) and a reference arm on which is arranged a reflecting device (134, 135); and wherein

- the splitting element (130) has a reflection coefficient and a transmission coefficient that are not equal, such that the proportion of the optical power of the incident light waves sent to the object arm is strictly higher than the proportion of the optical power of the light waves sent to the reference arm;
- the reflecting device is suitable for reflecting the incident light waves in a direction that is different from the direction of incidence, and
- the interferometer is suitable for producing, when the sample is placed on the object arm of the interferometer, at each point of an imaging field, interference between a reference wave obtained by reflection of incident light waves from an elementary surface of the reflecting device corresponding to said point of the imaging field and an object wave obtained by backscatter of incident light waves by a voxel of a slice of the sample at a given depth, said voxel corresponding to said point of the imaging field;

- a detection channel (102) comprising a device for acquiring two-dimensional images (121), and wherein:

o the device for acquiring two-dimensional images is positioned in a conjugate plane of the reflecting device (134, 135) and suitable for acquiring two-dimensional interferometric signals resulting from the interference produced at each point of the imaging field;

o the illumination channel (101) and the detection channel (102) comprise a common channel comprising said entrance face of the splitting element (130), and are separated by a reflecting element (151, 152), which is positioned in proximity to an image focal plane of said first objective (112, 138), or in proximity to a conjugate plane of said image focal plane of the first objective, said reflecting element being suitable for letting pass, on the detection channel, at least one portion of the light waves reflected by the reflecting device of the reference arm and for letting pass, on the illumination channel, at least one portion of the incident light waves;

- a processing unit (104) configured to calculate an image of the sample from said two-dimensional interferometric signals.

2. The imaging system according to claim 1, wherein the reflection coefficient and the transmission coefficient of the splitting element are such that at least 90% of the optical power of the light waves incident on the splitting element are sent toward the object arm.

3. The imaging system according to any one of the preceding claims, wherein the reflecting element comprises a mirror that is inclined with respect to the optical axis of the first objective, and which is apertured for the passage of the incident light waves, the dimensions of the aperture being sufficiently small to not block all of the light waves reflected onto the detection channel.

4. The imaging system according to any one of claims 1 or 2, wherein the reflecting element comprises a mirror that is inclined with respect to the optical axis of the first objective in order to deflect the incident light waves toward the common channel, and that has dimensions that are sufficiently small to not block all of the waves reflected onto the detection channel.

5. The imaging system according to any one of the preceding claims, wherein the reflecting device comprises a reflective diffraction grating, for example a blazed grating.

6. The imaging system according to any one of claims 1 to 4, wherein the reflecting device comprises an inclined mirror.

7. The imaging system according to any one of the preceding claims, wherein the interferometer (103) is a Michelson interferometer, said first objective (112) being arranged on the common channel of the illumination and detection channels (101, 102).

8. The imaging system according to any one of claims 1 to 6, wherein the interferometer (103) is a Linnik interferometer and comprises said first objective (138) on the reference arm of the interferometer, and a second objective on the object arm of the interferometer.

9. The imaging system according to claim 8, wherein said objectives are microscope objectives.

10. A full-field interferential imaging method for imaging a sample (S) by means of an imaging system according to any one of claims 1 to 9, comprising:

- placing the sample (S) on the object arm of the interferometer (103);
- producing, by means of said interferometer, for each point of an imaging field, interference between a reference wave obtained by reflection of incident light waves from the reflecting device of the reference arm of the interferometer, said elementary surface corresponding to said point of the imaging field, and an object wave obtained by backscatter of the incident wave by a voxel of a slice of the sample at a given depth, said voxel corresponding to said point of the imaging field;
- acquiring, for at least one value of the optical path difference between the object arm and the reference arm, at least one two-dimensional interferometric signal resulting from interference for each point of the imaging field;
- calculating an image of the sample from said two-dimensional interferometric signals.

ART ANTERIEUR

FIG.1A

FIG.1B

ART ANTERIEUR

20

FIG.2A

FIG.2B

FIG.3A

FIG.3B

FIG.4

400

121

122

113

111

152

104

114

115

130A

138

134

103

130

P2

137

P1

133

S

136

FIG.5

601

602

603

FIG.6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Full-field optical coherence tomography. **A. DUBOIS ; C. BOCCARA.** Optical Coherence Tomography - Technology and Applications. Springer, 2009
- **E. AUKSORIUS et al.** Dark-field full-field optical coherence tomography. *Optics Letters,* 2015, vol. 40 (14

- **E. AUKSORIUS et al.** Fingerprint imaging from the inside of a finger with full-field optical coherence tomography. *Biomédical Optics Express,* 2015, vol. 6 (11